# EUROPEAN PATENT SPECIFICATION

(11) **EP 3 294 208 B1**
(45) Date of publication and mention of the grant of the patent: **13.12.2023**
(21) Application number: 16796967.4
(22) Date of filing: 12.05.2016
(51) Int. Cl.: A61F 2/01

(54) **TETHERED FILTER ASSEMBLIES**
ANGEBUNDENE FILTERANORDNUNGEN
ENSEMBLES DE FILTRES ANCRÉS

(30) Priority: 15.05.2015 US 201562162570 P
(43) Date of publication of application: 21.03.2018
(73) Proprietor: Teleflex Medical Incorporated, Morrisville, North Carolina 27560 (US)
(72) Inventor: KORKUCH, Christopher N., Morrisville, North Carolina 27560 (US); KUEHN, Jeffrey P., Morrisville, North Carolina 27560 (US); TREXLER, Wade K., Morrisville, North Carolina 27560 (US); BOHN, Richard E., Morrisville, North Carolina 27560 (US); MURPHY, Aileen, Morrisville, North Carolina 27560 (US); ROBERTSON, Taylor J., Morrisville, North Carolina 27560 (US)
(74) Representative: Atout PI Laplace
(86) International application number: PCT/US2016/031977
(87) International publication number: WO 2016/186939

(56) References cited:
- US-A1- 2001 041 909
- US-A1- 2004 093 012
- US-A1- 2004 153 118
- US-A1- 2004 153 118
- US-A1- 2007 100 373
- US-A1- 2010 087 908
- US-A1- 2014 046 358
- US-B1- 6 939 361
- US-B2- 8 647 359

## Description

### Technical Field

This application relates generally to filter assemblies for use during medical procedures and, more specifically, to tethered mesh filters for capturing occlusive material.

### Background

During various vascular procedures, particles such as embolic material may become dislodged from the walls of a blood vessel. For example, during an atherectomy, plaque in an artery may be ground using a macerator, which may cause particles of plaque to break away from the arterial walls and enter the bloodstream. If the dislodged particles are not caught and removed from the bloodstream, then the particles may cause a blockage or embolism elsewhere. Depending on the size of the particles and where the blockage occurs, the blockage may create serious complications. For instance, if the blockage happens in the heart, then a sudden heart attack or cardiac arrest may occur.

A filter may be used to reduce the risk of a blockage resulting from a dislodged particle. The filter may be used to capture and trap potentially occlusive material in the bloodstream during a medical procedure. The filter may be positioned in the lumen of a blood vessel downstream from the location of the medical procedure. The filter may be designed to capture particles that may be sufficiently large to cause a blockage while allowing smaller particles and blood to flow through. After the procedure is finished, the filter may be removed from the vessel with the captured particles.

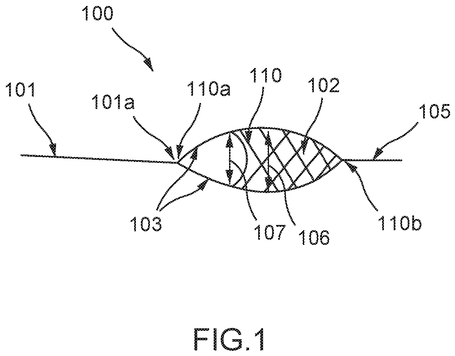

FIG. 1 provides one example of a filter assembly. A tethered filter assembly 100 has a filter wire 101 with a proximal end (not depicted) and a distal end lOla. The filter assembly 100 further has a filter 110 with a proximal end 110a and a distal end 110b. The proximal end 110a of the filter 110 may be coupled to the distal end 101 a of the filter wire 101, and the distal end 110b of the filter 110 may be coupled to a flexible member 105. The flexible member 105 may be formed from a less rigid material than the filter wire 101 or the filter 110. The distal end 110b of the filter 110 may have a degree of freedom at least in an axial direction of the filter wire 101.

The filter 110 may include a mesh 102 with a major diameter 106. The mesh 102 may include mesh members that are spaced apart as to create a series of voids. The voids may be sized to collect or pass certain sizes of particles such as occlusive material within the blood stream The filter 110 may further include at least two support members 103 that together open into an opening diameter 107. The opening diameter 107 is smaller than the major diameter 106 of the mesh 102. The opening diameter 107 may be disposed at a point where the support members 103 attach to the mesh 102.

The filter 110 may also be expandable. Prior to placement within a vessel, the filter 110 may be compressed and enclosed in a sleeve or sheath. During deployment, the filter 110 may be moved to a position within the vessel and moved relative to the sleeve, allowing the filter 110 to expand to fill the diameter of the vessel. Once expanded, the mesh 102 of the filter 110 may be in substantial contact with the inner surface of the vessel. The mesh 102 may operate to capture particles within the blood flowing through the vessel.

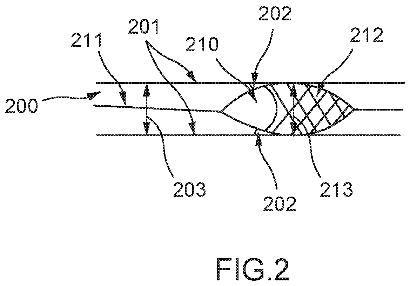

While existing filter assemblies such as the filter assembly 100 may trap certain particles that are loose in the bloodstream, they have several drawbacks. In FIG. 2, a filter assembly with a filter 210 and a filter wire 211 is placed in a vessel of a patient. The vessel has a vessel lumen 200 and a vessel wall 201. The filter 210 may be sized to fit within the vessel such that a mesh 212 of the filter 210 comes into substantial contact with the inner surface of the vessel wall 201. As such, in its expanded state (*e.g.,* a free state), the filter 210 may have a major diameter 213 that increases until the mesh 212 comes into contact with vessel wall 201.

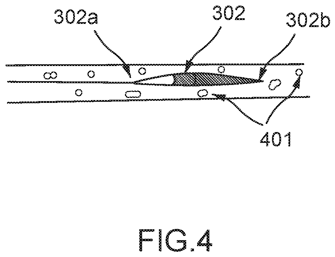

The proximal end (not depicted) of the filter wire 211 may be located outside of the patient, and a distal end of the filter wire 211 may be located within the vessel lumen 200. As blood carrying occlusive material flows through the filter 210, a portion of the occlusive material (not depicted) may be captured by the mesh 212 of the filter 210. Due to the conical shape of the filter 210, however, a space may exist between a proximal end of the mesh 212 and the vessel wall 201 where there may be a buildup 202 of occlusive material. Accordingly, when the filter 210 is removed from the vessel, occlusive material buildup 202 may be released back into the bloodstream, as shown on FIG. 4. Such occlusive material may then become lodged downstream in another blood vessel.

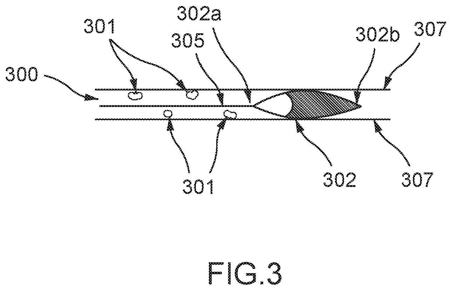

When a filter assembly is not held against the inside of a vessel wall, occlusive material may also bypass the filter assembly and pose a risk of blockage. As depicted in FIG. 3, when a filter assembly is properly deployed within a vessel, a filter 302 of the filter assembly is in substantial contact with a vessel wall 307 of the vessel. In such a state, the filter 302 may capture occlusive material 301 in the bloodstream that travels through a lumen 300 of the vessel.

A proximal end 302a of the filter 302 may be attached to a filter wire 305, and a distal end 302b of the filter 302 may be unconstrained. When there is an abrupt pull on the filter wire 305 in the proximal direction, the filter 302 may collapse (see FIG. 4) because the axially unconstrained distal end 302b of the filter 302 may not immediately travel with the filter wire 305 and the proximal end 302a of the filter 302. Upon collapsing, the filter 302 may no longer be in contact with the vessel wall 307, and occlusive material 401 may travel past the filter 302 in the area between the filter 302 and the vessel wall 307.

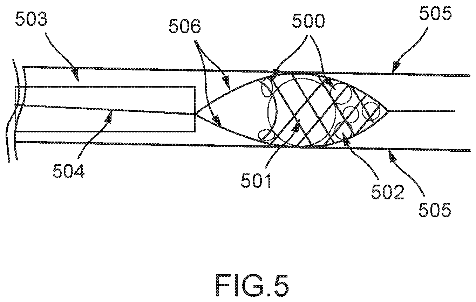

Another problem may arise when the occlusive material that is captured by the filter assembly is too large to allow for removal through the medical device 503. For example, as shown in FIG. 5, a filter 502 deployed within a vessel wall 505 may capture occlusive material with varying sizes. A medical device 503, such as an aspiration catheter, may be introduced over a filter wire 504 coupled at its distal end to filter 502 to vacuum the occlusive material. But the medical device 503 may not be able to remove all of the occlusive material in the filter 502. For one, the medical device 503 may have an opening that is too small to remove large occlusive material such as particle 501 depicted in FIG. 5. For another, the medical device 503, which is introduced over the filter wire 504, may be blocked by the support members 506 from entering the filter 502 to remove all of the occlusive material. Thus, smaller occlusive material such as particles 500 that can fit in the opening of the medical device 503 but are positioned at a far end of the filter 502 may not be fully removed from the vessel. Moreover, the medical device 503 may not be able to reach smaller particles 500 that are trapped behind the larger particle 501.

To break up the particle 501 into smaller particles that can be vacuumed out by the medical device 503, a mechanical thrombectomy macerator may be introduced into the vessel. But the mechanical thrombectomy macerator may also be blocked by the support members 506 from entering the filter 502 to break down particle 501.

When an acceptable amount of occlusive material is not removed from the filter 502, various complications may arise. For example, when the filter 502 is being removed from the patient, a portion of the occlusive material may become loose and fall out of the filter 502, increasing the risk of pulmonary embolism. If the filter 502 is engorged with occlusive material during removal, then the scraping of the sides of the filter 502 against the vessel wall 505 may increase the risk of hematoma and further clotting. Furthermore, if the filter 502 cannot be removed from the vessel through existing access channels due to its engorged size, then the filter 502 may create procedural inefficiencies and block off access for devices that need to be utilized in later stages of a procedure.

US2014046358 discloses a dual net vascular filtration device comprises a central frame, a proximal filter net attached to a proximal end of the central frame, and a distal filter net attached to a distal end of the central frame. Upon deployment, the distal filter net can be configured to evert into the proximal filter net.

Accordingly, a new tethered mesh filtered is needed to overcome these limitations of known tethered mesh filters.

### Summary

The present disclosure overcomes the foregoing and other shortcomings and drawbacks of tethered mesh filters. While the invention will be described in connection with certain embodiments, it will be understood that the invention is not limited to these embodiments. On the contrary, the invention includes all alternatives, modifications and equivalents as may be included within the scope of the present invention which is defined by the filter assembly of claim 1

According to an aspect, a filter assembly includes a filter. The filter may include a proximal portion comprising support members a distal portion comprising a mesh. The proximal portion has a first major diameter and the distal portion has a second major diameter. In a first state, the second major diameter may be less than the first major diameter.

According to another aspect, a filter assembly includes a filter wire, a tubular member comprising a lumen, and a filter. The filter may include a proximal portion comprising support members a distal portion comprising a mesh. The tubular member may be attached to a proximal end of the filter, and the filter wire may be attached to a distal end of the filter. The filter has a major diameter that may change depending on the particular state of the filter.

According to yet another aspect, a filter assembly includes a filter having a first portion comprising a first mesh, a second portion comprising support members, and a third portion comprising a second mesh. The first portion has a first major diameter, the second portion has a second major diameter, and the third portion has a third major diameter. The filter is configured to be in a first state, where the first portion is disposed proximal to the second portion and the third portion, where the second portion is disposed proximal to the third portion, and where the first major diameter, the second major diameter, and third major diameter equal one another.

The above and other objects and advantages of the present invention shall be made apparent from the accompanying drawings and the description thereof.

### Brief Description of the Drawings

In order that the disclosure may be readily understood, aspects of the disclosure are illustrated by way of examples in the accompanying drawings.
FIG. 1 is a sectional view of a filter assembly configured to capture occlusive material in a vessel.
FIG. 2 is a sectional view of a vessel depicting a filter assembly deployed in the vessel where a side of the filter assembly is in contact with a wall of the vessel.
FIG. 3 is a sectional view of a vessel depicting occlusive material travelling towards a filter assembly deployed in the vessel.
FIG. 4 is a sectional view of a vessel depicting occlusive material travelling past a filter assembly deployed in the vessel.
FIG. 5 is a sectional view of a vessel depicting a medical device introduced into the vessel to remove occlusive material captured by a filter assembly.
FIG. 6 is a sectional view of a filter assembly configured to capture occlusive material in a vessel according to a first exemplary aspect of the invention.
FIG. 7 is a sectional view of a vessel depicting the filter assembly of FIG. 6 deployed in the vessel.
FIG. 8 is a sectional view of a filter assembly configured to capture occlusive material in a vessel according to a second exemplary aspect of the disclosure. The filter assembly is depicted in a first state in FIG. 8.
FIG. 9 is a sectional view of the filter assembly of FIG. 8 in a second state.
FIG. I 0 is a sectional view of the filter assembly of FIG. 8 in a third state.
FIG. 11 is a perspective view of a locking device configured to lock a position of a sheath of a filter assembly relative to a position of a wire of a filter assembly according to aspects of the disclosure.
FIG. 12 is a sectional view of a filter assembly configured to capture occlusive material in a vessel according to a third exemplary aspect of the disclosure. The filter assembly is depicted in a first state in FIG. 12.
FIG. 13 is a sectional view of the filter assembly of FIG. 12 in a second state.
FIG. 14 is a sectional view depicting the filter assembly of FIG. 12 deployed in the second state and positioned to capture occlusive material in the vessel.
FIG. 15 is an image of a filter assembly configured to capture occlusive material in a vessel according to a fourth exemplary aspect of the disclosure.
FIGS. 16, 17, and 18 are images depicting the filter assembly of FIG. 15 in a plurality of different states.
FIG. 19 is a sectional view of a filter assembly configured to capture occlusive material in a vessel according to a fifth exemplary aspect of the disclosure. The filter assembly is depicted in a first state in FIG. 19.
FIG. 20 is a sectional view of the filter assembly of FIG. 19 in a second state.
FIG. 21 is a sectional view of a filter assembly configured to capture occlusive material in a vessel according to a sixth exemplary aspect of the disclosure.
FIG. 22 is a sectional view of a vessel depicting the filter assembly of FIG. 21 deployed in the vessel in a first state.
FIG. 23 is a sectional view of a vessel depicting the filter assembly of FIG. 21 deployed in the vessel in a second state and positioned to capture occlusive material in the vessel.
FIG.24 is a sectional view of a vessel depicting a medical device introduced into the vessel to remove occlusive material captured by the filter assembly of FIG. 21.
FIG. 25 is a sectional view of a vessel depicting the filter assembly of FIG. 21 deployed in the vessel in a third state and positioned to capture occlusive material in the vessel.

Aspects of these exemplary tethered filter assemblies are described with reference to the drawings, in which like reference numerals refer to like parts throughout.

### Detailed Description

Systems and methods disclosed herein provide a tethered mesh filter that overcomes many of the drawbacks associated with existing filter assemblies. Systems and methods disclosed herein provide filter assemblies for capturing occlusive material that include a mesh and support members, where a major diameter of the filter may be disposed along the support members. The mesh and the support members are coupled to enable more than one degree of freedom of movement.

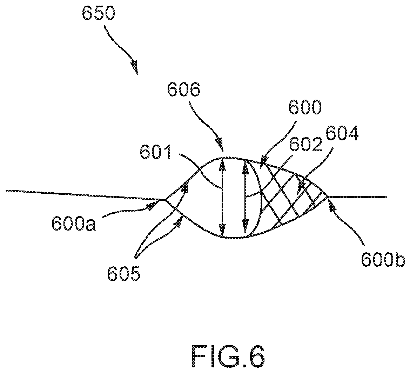

Referring now to FIG. 6, a first exemplary aspect according to the invention is depicted. A filter assembly 650 includes a tethered filter 600. The tethered filter 600 includes a mesh 604 and support members 605. The mesh 604 has an opening diameter 602, and the support members 605 have a major diameter 601. In a free or expanded state, the major diameter 601 of the support members 605 is greater than the opening diameter 602 of the mesh 604.

The tethered filter 600 may have a proximal end 600a and a distal end 600b. Support members 605 may be shaped with a bend at point 606. Specifically, the support members 605 may contact one another at the proximal end 600a of tethered filter 600 and extend outwards from a central axis running along the length of the tethered filter 600 until the bend point 606. At the bend point 606, the support members 605 extend back towards the central axis of the tethered filter 600. Accordingly, the major diameter 601 of the support members 605 is located at the bend point 606.

The mesh 604 may be shaped conically and extend inwards from its point of contact with the support members 605 until the distal end 600b of the tethered filter 600. Thus, the opening diameter 602 of the mesh 604 is located at a proximal end of the mesh 604.

The tethered filter 600 is expandable. Prior to placement within a vessel, the tethered filter 600 may be compressed and enclosed in a sheath (not shown in FIG. 6). During deployment, the tethered filter 600 may be moved to a position within the vessel and the sheath may be removed, allowing the tethered filter 600 to expand to the diameter of the vessel.

The tethered filter 600 may be sized to fit within the vessel such that the mesh 604 is in substantial contact with the vessel. To enable contact with the mesh 604, the major diameter 601 of the support members 605 and the opening diameter 602 of the mesh 604 may be equal to or greater than an inner diameter of the vessel.

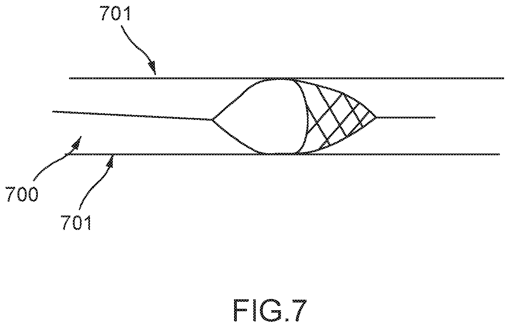

The vessel depicted in FIG. 7 has a vessel wall 701 and a vessel lumen 700. When the filter 600 is deployed in the vessel lumen 700, the filter 600 may have tangential and substantial contact with an inner surface of the vessel wall 701. Because the contact of filter 600 with the inner surface of the vessel wall 701 begins at a point along the support members 605 and further upstream from mesh 604, space does not exist between a proximal end of mesh 604 and the vessel wall 701. Accordingly, occlusive material may not build between the filter 600 and the vessel wall 701, which reduces the risk of such material escaping back into the bloodstream while the tethered filter 600 is removed from the vessel.

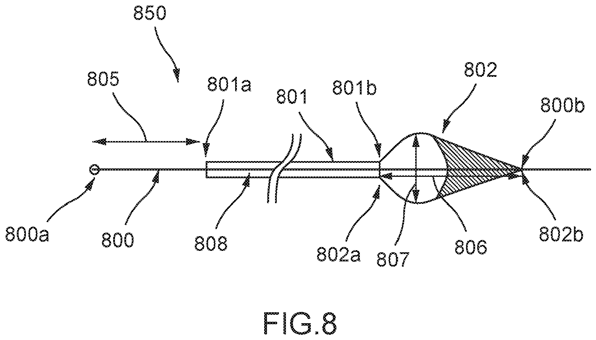

According to a second exemplary aspect shown in FIG. 8, a filter assembly 850 includes a wire 800, a tube 801, and a tethered filter 802. The wire 800 has a proximal end 800a and a distal end 800b; the tube 801 has a proximal end 801a and a distal end 801 b with a lumen 808 in between the proximal end 801a and the distal end 801 b; and the tethered filter 802 has a proximal end 802a and a distal end 802b. The wire 800 may extend from outside of a patient through the lumen 808 of the tube 801 to the distal end 802b of the tethered filter 802. The proximal end 802a of the tethered filter 802 may be coupled to the distal end 801 b of the tube 801. The distal end 802b of the tethered filter 802 may be coupled to the distal end 800b of the wire 800. Accordingly, axial movement of the tube 801 relative to the wire 800 may change the shape of the tethered filter 802.

The tethered filter 800 is shown in a first position in FIG. 8. In the first position, the tethered filter 800 may be unconstrained or in a free state. The wire 800 may have a first length of exposed wire 805. The tethered filter 802 may have a first length 806 and a first opening diameter 807. The opening diameter 807 of the tethered filter 802 may be equal to or greater than a diameter of a target vessel.

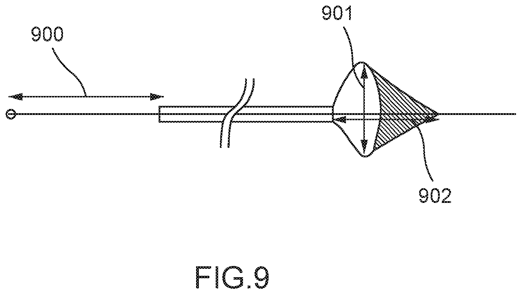

FIG. 9 shows the tethered filter 800 in a second position. In the second position, the tethered filter 802 may be constrained to having a second length 902 that is less than the first length 806 and a second opening diameter 901 that is greater than the first opening diameter 807. The wire 800 may be pulled in the proximal direction or the tube 801 may be pushed in the distal direction to constrain the tethered filter 802 to the second position. The wire 800 may have a second length 900 of exposed wire 800 that is greater than the first length 805 of the exposed wire 800.

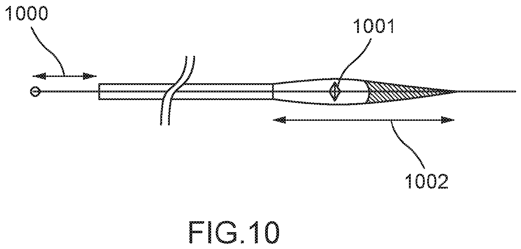

FIG. 10 shows the tethered filter 800 in a third position. In the third position, the tethered filter 802 may be constrained to having a third length 1002 that is greater than the first length 806 and a third opening diameter 1001 that is less than the first opening diameter 807. The wire 800 may be pushed in the distal direction or the tube 804 may be pulled in the proximal direction to constrain the tethered filter 802 to the third position. The wire 800 may have a third length of exposed wire 1000 that is less than the first length 805 of exposed wire 800.

When tethered filter 802 is in the unconstrained position shown in FIG. 8, the tethered filter 802 may function similar to the filter 600 shown in FIG. 6. When the tethered filter 802 is constrained into a different shape, such as the shapes shown in FIGS. 9 and 10, it may have enhanced or reduced vessel contact, respectively.

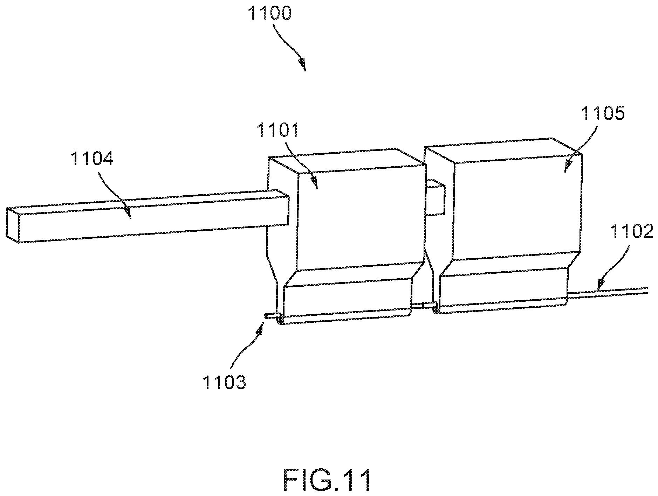

In some aspects, the tethered filter 802 may be locked into a particular shape using locking device 1100 shown in FIG. 11. The locking device 1100 may include a tube coupler 1105 and a wire coupler 1101. The tube coupler 1105 may be selectively coupled to a tube 1102, and the wire coupler 1101 may be selectively coupled to the wire 1103. The tube 1102 may correspond to the tube 801, and the wire 1103 may correspond to the wire 800.

The wire coupler 1101 may slide along an axis of an extending member 1104 of the tube coupler 1105. The wire coupler 1101 and the tube coupler 1105 may be selectively unlocked or locked in their respective positions. Specifically, the wire coupler 1101 and the tube coupler 1105 may be locked in position with respect to one another in a number of different ways. For example, the wire coupler 1101 may be locked in position along the extending member 1104 using a friction or interference fit between the wire coupler 1101 and the extending member 1104. As another example, the wire coupler 1101 may be held in position along the extending member 1104 using a clamping lever or other lever design. As yet another example, the wire coupler 1101 may be locked at a position along the extending member 1104 via interlocking grooves or notches that lock into position at discreet points along the extending member 1104.

The tube coupler 1105 and the wire coupler 1101, when selectively coupled to the tube 1102 and the wire 1103, respectively, and selectively locked in position relative to one another, may enable the constraining and unconstraining of the filter 802. Moreover, when selectively decoupled from the tube 1102 and the wire 1103, the tube coupler 1105 and the wire coupler 1101 may enable a medical device to pass over the tube-wire assembly without any obstruction.

When the tethered filter 802 is locked into a particular shape, the tethered filter 802 may maintain the same degree of contact with the vessel regardless of any abrupt movement of the wire 800, as any movement of the wire 800 results in an equal movement of tube 801 and has no effect on the shape of the filter 802. By maintaining the same degree of contact regardless of any abrupt movement of wire 800, occlusive material would not flow past the tethered filter 802 via space between the tethered filter 802 and a vessel wall. Accordingly, complications associated with pulmonary embolism from occlusive material travelling pass the tethered filter 802 may be minimized.

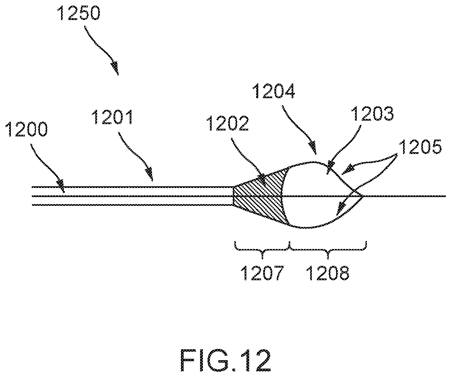

A filter assembly according to a third exemplary aspect is shown in FIG. 12. Filter assembly 1250 includes a wire 1200, a tube 1201, and a tethered filter 1203. The tethered filter 1203 has a mesh 1202 and support members 1205. The tethered filter 1203 has a major diameter located at a point 1204 along support members 1205.

The mesh 1202 is located in a proximal region 1207 of the tethered filter 1203, and the support members 1205 are located in a distal region 1208 of the tethered filter 1203. Thus, as depicted in FIG. 12, the proximal end of the mesh 1202 located in the proximal region 1207 may be coupled to the distal end of the tube 1201, and the distal end of the support members 1205 located in the distal region 1208 may be coupled to the distal end of wire 1200. Furthermore, the distal end of the mesh 1202 may be coupled to the proximal end of support members 1205.

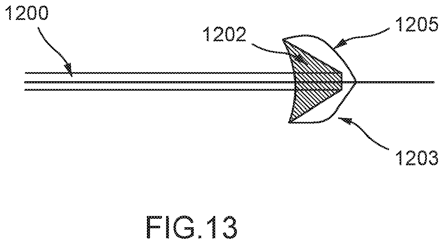

The filter assembly 1250 may enable a first degree of freedom of the tethered filter 1203 in the coupling of the proximal region 1207 (including the mesh 1202) to the distal end of the tube 1201. The filter assembly 1250 may further enable a second degree of freedom of the tethered filter 1203 in the coupling of the proximal region 1207 (including the mesh 1202) to the distal region 1208. Such coupling may enable an inversion of the tethered filter 1203, as shown in FIG. 13, where the mesh 1202 may fold inside of distal region 1208.

In FIG. 12, the tethered filter 1203 is shown in a first position. In the first position, the tethered filter 1203 may be in an unconstrained or free state. In FIG. 13, the tethered filter 1203 is shown in a second position. In the second position, the tethered filter 1203 may be constrained into a state where the mesh 1202 has folded inside distal region 1208.

One method of constraining the tethered filter 1203 to the second position may be to pull the wire 1200 in the proximal direction. The distal region 1208, which is coupled to the wire 1200 at its distal end, may then move with the wire 1200 in the proximal direction. As the distal region 1208 begins to move in the proximal direction, the mesh 1202 may begin to invert or fold along itself. When the wire 1200 is pulled a maximum amount in the proximal direction, the mesh 1202 may be fully inverted and positioned within the support members 1205.

A second method of constraining the tethered filter 1203 to the second position may be to push tube 1201 in the distal direction. As the tube 1207 moves in the distal direction, the mesh 1202 of the proximal region 1207 may again invert or fold along itself.

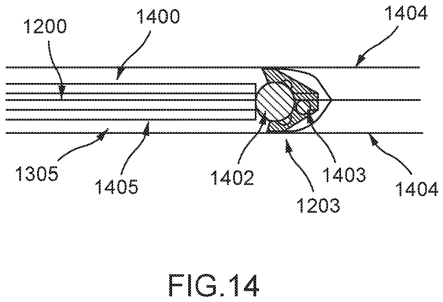

As shown in FIG. 14, when the tethered filter 1203 is deployed within a lumen 1305 of a vessel 1400 and transitioned into its fully inverted state, the tethered filter 1203 may make substantial contact with a vessel wall 1404. Specifically, the support members 1205 of the tethered filter 1203 may be in substantial contact with the vessel wall 1404. Mesh 1202 may also make substantial contact with vessel wall 1404. As deployed, the tethered filter 1203 may capture occlusive material within the bloodstream such as small occlusive material 1403 and large occlusive material 1402.

To remove the occlusive material captured by the tethered filter 1203, a medical device 1405, such as an aspiration catheter or a mechanical thrombectomy macerator, may be introduced into the vessel 1400 over the wire 1200. The tethered filter 1203 in its inverted position may allow the medical device 1405 to contact the occlusive material that has been captured. Thus, where the medical device 1405 is a mechanical thrombectomy macerator, the mechanical thrombectomy macerator may be used to break down the large occlusive material 1402 into smaller particles. And, where the medical device 1405 is an aspiration catheter, the aspiration catheter may be used to effectively remove the small occlusive material 1403 from tethered filter 1203.

In certain aspects, the aspiration catheter may be introduced into the vessel 1400 using an additional wire (not depicted). The aspiration catheter first may be inserted over the wire 1200 and moved along the wire 1200 until a distal end of the aspiration catheter is proximate to or in contact with the tethered filter 1203. The additional wire may then be inserted into the aspiration catheter until a distal end of the additional wire also proximate to or in contact with the tethered filter 1203. The aspiration catheter may then be removed and reinserted over the additional wire in place of the wire 1200 until its distal end once again is proximate to or in contact with the tethered filter 1203. The additional wire may then be removed. The aspiration catheter, now without the wire 1200 or the additional wire disposed within it, may more effectively remove occlusive material, such as occlusive material 1403, from the tethered filter 1203.

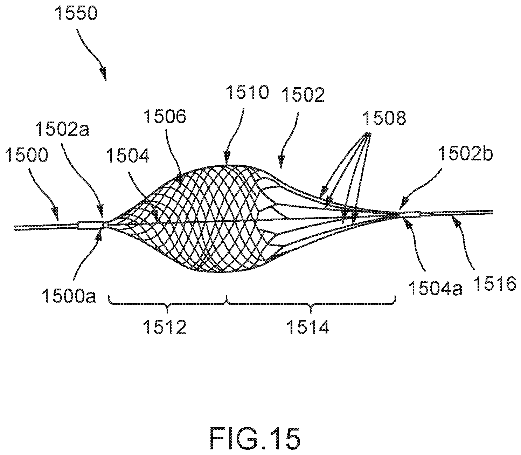

According to a fourth exemplary aspect shown in FIG. 15, a filter assembly 1550 includes a wire 1504, a tube 1500, and a tethered filter 1502. The tethered filter 1502 has a proximal region 1512 and a distal region 1514. The proximal region 1512 includes a series of members creating a series of voids. The voids create a mesh 1506. The distal region 1514 includes support members 1508. The tethered filter 1502 has a major diameter located at a point 1510 along the mesh 1506.

The wire 1504 may extend out of tube 1500 on a proximal end (not depicted) and a distal end 1500a. A proximal end 1502a of the tethered filter 1502 may be coupled to a distal end 1500a of the tube 1500. A distal end 1502b of the tethered filter 1502 may be coupled to a distal end 1504a of the wire 1504. The distal end 1502b of the tethered filter 1502 may also be coupled to another wire 1516. The wire 1516 may have greater flexibility than the wire 1504.

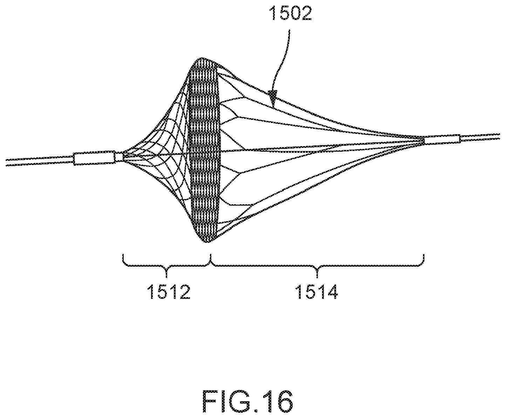

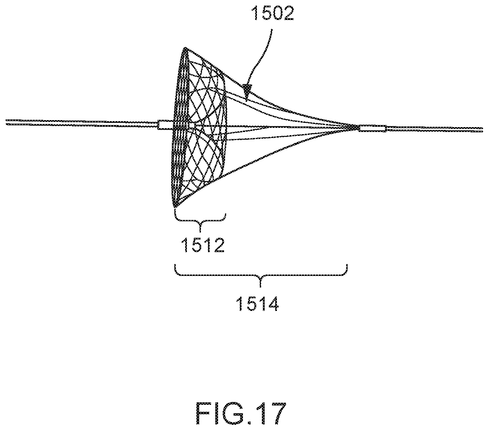

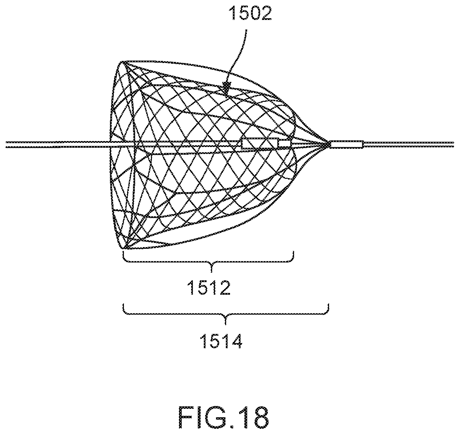

In FIG. 15, the tethered filter 1502 is shown in a first position. In the first position, the tethered filter 1502 may be unconstrained or in a free state. In FIGS. 16 through 18, the tethered filter 1502 is changed from its unconstrained position into an inverted position shown in FIG. 18. In some aspects, the tethered filter 1502 may be changed into the inverted position by moving (or shifting) the wire 1504 in a proximal direction, and in other aspects, the tethered filter 1502 may be changed into the inverted position by moving the tube 1500 in a distal direction.

As the tethered filter 1502 is changed from its unconstrained position into the inverted position, the mesh 1506 may first compress in length and then expand in length. In the inverted position shown in FIG. 18, the mesh 1506is located inside the distal region 1514.

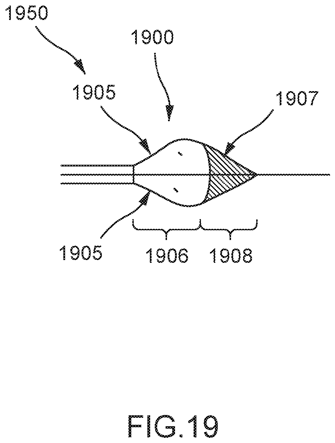

FIG. 19 shows a filter assembly 1950 according to a fifth aspect. The filter assembly 1950 includes a tethered filter 1900. The tethered filter 1900 has support members 1905 and a mesh 1907. The support members 1905 are located in a proximal region 1906 of the tethered filter 1900, and the mesh 1907 is located in a distal region 1908 of the tethered filter 1900.

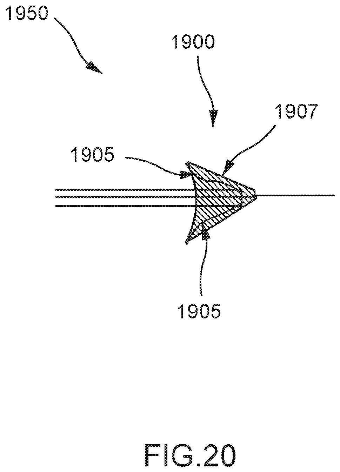

The tethered filter 1900 is shown in a free or unconstrained state in FIG. 19. In FIG. 20, the tethered filter 1900 is changed into a second state where the support members 1905 are located inside the mesh 1907. The support members 1905 within the mesh 1907 may block access to a portion of the inside of mesh 1907. Such a configuration may be desirable for use with certain medical devices such as, for example, a light or a sensor, where it is desirable to position the medical device a certain distance away from a distal end of the mesh 1907.

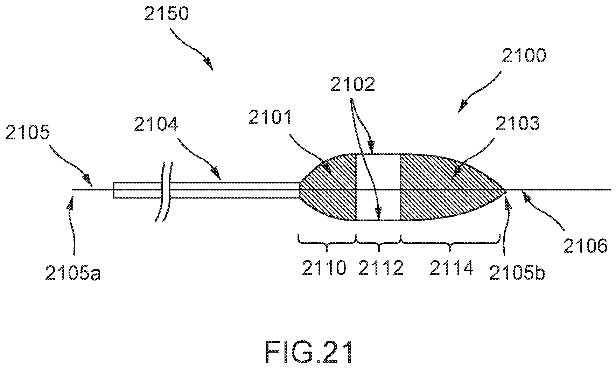

FIG. 21 depicts a sixth exemplary aspect, where a filter assembly 2150 includes a tethered filter 2100, a tube 2104, and a wire 2105. The tethered filter 2100 includes a first mesh 2101, a second mesh 2103, and support members 2102. The first mesh 2100 may be located in a first proximal region 2110 of the filter 2100; the second mesh 2100 may be located in a distal region 2114 of the filter 2100; and the support members 2102 may be located in a second proximal region 2112 of the filter 2100. The second proximal region 2112 may be distal to first proximal region 2110, and the distal region 2114 may be distal to both the first proximal region 2110 and the second proximal region 2112.

The first mesh 2101 may be coupled to the tube 2104 at a proximal end and the support members 2102 at a distal end. The support members 2102 may be coupled to the second mesh 2103 at a distal end.

The wire 2105 may have a proximal end 2105a and a distal end 2105b. The proximal end 2105a may be located outside of the tube 2104. The distal end 2105b may be coupled to a distal end of the second mesh 2103. The filter assembly 2150 may also include an additional wire 2106 that is more flexible than the wire 2105. A proximal end of the additional wire 2106 may be coupled to a distal end of the second mesh 2103.

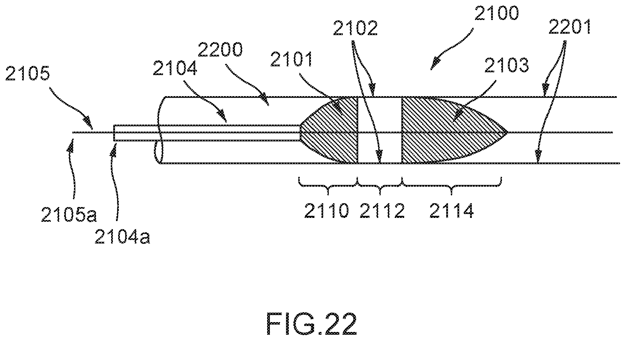

As shown in FIG. 21, the tethered filter 2100 is in a first position. In the first position, tethered filter 2100 may be in an unconstrained or free state. The filter assembly 2150 in its first position may be deployed and constrained in a vessel lumen 2200 within a patient's body, as shown in FIG. 22. The first mesh 2101, the support members 2102, and the second mesh 2103 are in substantial contact with a vessel wall 2201.

As deployed, a proximal end 2104a of the tube 2104 may be located outside of the patient's body, and the proximal end 2105a of the wire 2105 may also be located outside of the patient's body. The proximal end 2105a of the wire 2105 may extend out of the proximal end 2104a of the tube 2104.

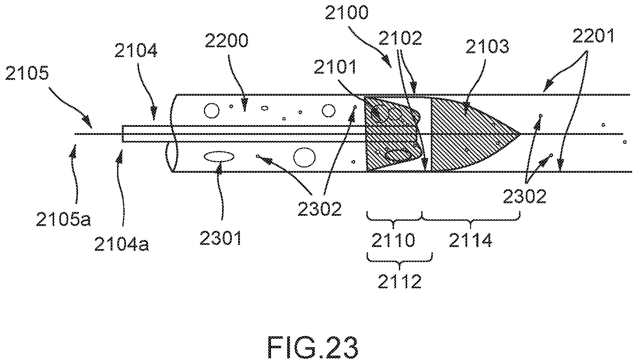

In FIG. 23, the tethered filter 2100 is deployed in the vessel lumen 2200 in a second position. In the second position, the tethered filter 2100 is constrained where at least a portion of the first proximal region 2110 is positioned within the second proximal region 2112 of the tethered filter 2100. One method of constraining the tethered filter 2100 to the second position is by moving the proximal end 2105a of the wire 2105 in a proximal direction or away from the proximal end 2104a of the tube 2104. Another method of constraining the tethered filter 2100 to the second position is by moving the proximal end 2104a of the tube 2104 in a distal direction away from the proximal end 2105a of the wire 2105.

When deployed in the second position, the proximal end of the first proximal region 2110, second proximal region 2112, and the proximal end of distal region 2114 are in substantial contact with vessel wall 2101. The first mesh 2101 may capture occlusive material of a first general shape and size 2301. Occlusive material of a second general shape and size 2302, which is smaller than the occlusive material of the first general shape and size 2301, may pass through tethered filter 2100 and continue through the blood stream.

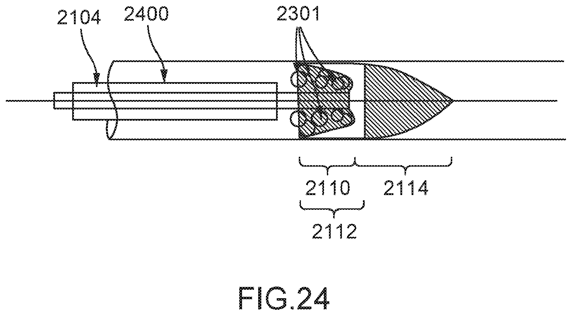

In FIG. 24, the occlusive material of the first general shape and size 2301 is shown captured within the first mesh 2101. Similar to the filter assembly 1250 shown in FIG. 12, the first mesh 2101 may permit a medical device 2400 to be inserted over tube 2104 into the proximal region 2110 of the tethered filter 2100. The medical device 2400 may be a mechanical thrombectomy device, which can be used to break down the occlusive material 2301 into a smaller size, or may be an aspiration catheter, which can be used to remove the occlusive material 2301 from the proximal region 2110.

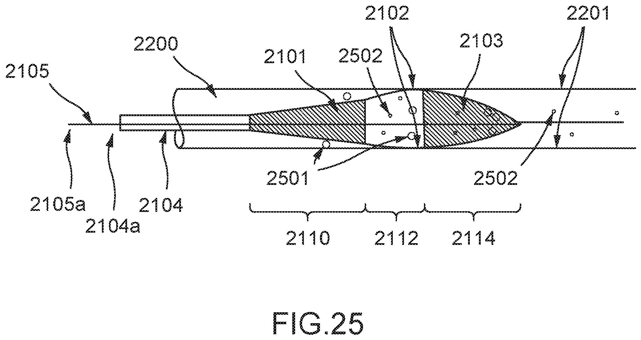

In FIG. 25, the tethered filter 2100 is shown in a third position within the vessel lumen 2200. In the third position, the proximal region 2110 and second proximal region 2112 are not in substantial contact with the vessel wall 2201, but the distal end of second proximal region 2112 and the proximal end of distal region 2114 are in substantial contact with vessel wall 2201. In addition, the first proximal region 2110 is not located within second proximal region 2112. In order to move the tethered filter 2100 into the third position, the proximal end 2105a of the wire 2105 may be moved in a distal direction or towards proximal end 2104a of tube 2104, or the tube 2104 may be moved in a proximal direction.

Any occlusive material, such as the occlusive material 2501 that was not removed from the first mesh 2101 may become loose when the tethered filter 2100 is moved from the second position into the third position because the first mesh 2101 may invert back from its constrained state into its unconstrained state, *i.e.,* change back into being in a convex shape as opposed to a concave shape. Nonetheless, because the proximal region 2110 and the proximal end of the second proximal region 2112 are not in contact with the vessel wall 2201, occlusive material of a minimum size 2501 may flow downstream from the first proximal region 2110 and the second proximal region 2112 into distal region 2114 and be caught by the second mesh 2103. Occlusive material of a smaller size 2502 may pass freely through the tethered filter 2100. The tethered filter 2100 may then be removed from the patient's body with the occlusive material 2501 within distal region 2114.

Systems and methods disclosed herein provide a filter assembly that includes a filter wire and a filter. The filter includes a proximal portion having a first major diameter, the proximal portion comprising a plurality of support members; and a distal portion having a second major diameter, the distal portion comprising a mesh. The filter wire is attached to a proximal end of the filter. And when the filter is in a unconstrained state, the second major diameter is less than the first major diameter. The support members of the filter may have a bend at a point corresponding to a location of the first major diameter in the unconstrained state. The filter may also have a deployed state, where the filter assembly is disposed within a lumen of a vessel, and where the second major diameter equals the first major diameter.

Systems and methods disclosed herein also provide a filter assembly that includes a filter wire, a tubular member, and a filter. The tubular member includes a wall and a lumen therein, where a first portion of the filter wire is disposed within the lumen of the tubular member and a second portion of the filter wire is disposed proximal to a proximal end of the tubular member. The filter includes a plurality of support members and a mesh, the filter having a major diameter. The proximal end of the filter is attached to a distal end of the tubular member, and a distal end of the filter is attached to a distal end of the filter wire. The filter is capable of changing from an unconstrained state to a first constrained state, where the major diameter of the filter in the first constrained state is greater than the major diameter of the filter in the unconstrained state, and where a length of the second portion of the filter wire in the first constrained state is greater than a length of the second portion of the filter wire in the unconstrained state.

In an aspect, the filter is further configured to change from the unconstrained state to a second constrained state, where is further configured to change from the unconstrained state to a second constrained state is less than the major diameter of the filter in the unconstrained state, and where a length of the second portion of the filter wire in the first constrained state is less than the length of the second portion of the filter wire in the unconstrained state.

In another aspect, the filter assembly further comprises a locking device. The locking device includes a first member configured to be attached to the filter wire, and a second member configured to be attached to the tubular member. The second member may include a sliding portion, where the first member is attached to the sliding portion of the second member, where the first member is configured to move along an axis defined by a length of the sliding portion, and where the first member is configured to be locked into a position along the axis defined by the length of the sliding portion. The first member may be locked into position using at least one of a frictional contact, a lever, and a notch. The locking device may be configured to be selectively detached from the filter wire and the tubular member.

Systems and methods disclosed herein also provide a filter assembly that includes a filter wire, a tubular member, and a filter, where the filter includes a proximal portion comprising a mesh and a distal portion comprising a plurality of support members. The tubular member includes a wall and a lumen, where a first portion of the filter wire is disposed within the lumen of the tubular member and a second portion of the filter wire is disposed proximal to a proximal end of the tubular member. A proximal end of the filter may be attached to a distal end of the tubular member, and a distal end of the filter may be attached to a distal end of the filter wire.

In an aspect, the filter is configured to change from an unconstrained state to a constrained state, where the proximal portion of the filter is disposed proximal to the distal portion of the filter in the unconstrained state, where the proximal portion of the filter is disposed within the distal portion of the filter in the constrained state, and where a length of the second portion of the filter wire in the constrained state is greater than a length of the second portion of the filter wire in the unconstrained state.

Systems and methods disclosed herein also provide a filter assembly that includes a filter wire, a tubular member, and a filter, where the filter includes a first proximal portion comprising a first mesh, a second proximal portion comprising a plurality of support members, and a distal portion comprising a second mesh. The tubular member includes a wall and a lumen therein, where a first portion of the filter wire is disposed within the lumen of the tubular member and a second portion of the filter wire is disposed proximal to a proximal end of the tubular member. A proximal end of the filter may be attached to a distal end of the tubular member, and a distal end of the filter may be attached to a distal end of the filter wire.

In an aspect, the filter is configured to change from an unconstrained state to a constrained state, where the first proximal portion of the filter is disposed proximal to the second proximal portion of the filter in the unconstrained state, where the first proximal portion of the filter is disposed within the second proximal portion of the filter in the constrained state, and where a length of the second portion of the filter wire in the constrained state is greater than a length of the second portion of the filter wire in the unconstrained state.

Systems and methods disclosed herein also provide a method of deploying a filter that includes inserting the filter into a vasculature, inserting the filter into a vasculature, moving the filter to a predetermined position within the vasculature, removing the sheath from the vasculature to deploy the filter in a first state, and changing the filter from the first state to a second state, where a proximal portion of the filter is disposed within a distal portion of the filter in the second state, and where the filter in the second state is configured to receive and hold a particle moving in the vasculature proximate to the predetermined position. In an aspect, inserting the filter into the vasculature may include inserting the filter into a sheath disposed within an access device, where at least a portion of the sheath and a portion of the access device are disposed within the vasculature.

Systems and methods disclosed herein also provide a method of removing a particle using a filter that includes inserting a catheter over a first wire of the filter to a first predetermined position, where the filter is disposed within the vasculature, where a distal end of the catheter in the first predetermined position is proximate to the filter, and where the catheter comprises a lumen. The method further includes inserting a second wire into the lumen of the catheter to a second predetermined position, where a distal end of the second wire in the second predetermined position is proximate to the filter. The method also includes removing the catheter; inserting the catheter over the second wire to a third predetermined position, where the distal end of the catheter in the third predetermined position is proximate to the filter; removing the second wire; and removing a particle held in the filter using the catheter. In an aspect, the distal end of the catheter in the third predetermined position can be disposed within the filter.

Systems and methods disclosed herein also provide a method of removing a particle using a filter that includes inserting an aspiration catheter over a wire of the filter to a predetermined position, where the filter is disposed within the vasculature, and where a distal end of the aspiration catheter in the predetermined position is disposed within the filter. The method further includes removing a particle held in the filter using the aspiration catheter.

Systems and methods disclosed herein also provide a method of removing a particle using a filter that includes inserting a thrombectomy device over a wire of the filter to a predetermined position, where the filter is disposed within the vasculature, and where a distal end of the thrombectomy device in the predetermined position is disposed within the filter. The method further includes separating the particle held in the filter into a plurality of smaller portions using the thrombectomy device.

While various aspects in accordance with the principles of the invention have been illustrated by the description of various embodiments, and while the embodiments have been described in considerable detail, they are not intended to restrict or in any way limit the scope of the invention to such detail. The various features shown and described herein may be used alone or in any combination. Additional advantages and modifications will readily appear to those skilled in the art. The invention in its broader aspects is therefore not limited to the specific details, representative apparatus and illustrative examples shown and described. Accordingly, departures may be made from such details without departing from the scope of the appended claims.

## Claims

1. A filter assembly (650) comprising:
a filter (600) comprising:
a proximal portion comprising a plurality of support members (605) having a major diameter (601); and
a distal portion comprising a mesh (604) having an opening diameter (602); and a filter wire attached to the filter,
**characterized in that** the opening diameter (602) is less than the major diameter (601) in a free, expanded state, and each of the plurality of support members (605) forms a bend (606) corresponding to the major diameter (601) by extending from the mesh (604) outwardly relative to a central axis and back toward the central axis proximally from the bend (606) to the filter wire.

2. The filter assembly of claim 1, further comprising a tubular member having a lumen.

3. The filter assembly of any one of claims 1-2, wherein the mesh comprises a conical shape.

4. The filter assembly of any one of claims 1-3, wherein the support members (605) comprise a plurality of wires (605).

5. The filter assembly of any one of claims 1-4, wherein the filter is radially compressible.

6. The filter assembly of any one of claims 1-5, wherein the filter is configured to fit in a lumen of a vessel in a compressed state, and the mesh (604) is configured to contact the vessel in the free, expanded state.

7. The filter assembly of any one of claims 1-6, wherein the support members (605) are configured to contact the vessel in the free, expanded state.

8. The filter assembly of any one of claims 1-7, wherein the second major diameter (602) is equal to the major diameter (601) when in a compressed state.

## Patentansprüche

1. Filteranordnung (650), umfassend:
einen Filter (600), umfassend:
einen proximalen Abschnitt, der eine Vielzahl von Stützelementen (605) mit einem Hauptdurchmesser (601) umfasst; und
einen distalen Abschnitt, der ein Gewebe (604) mit einem Öffnungsdurchmesser (602) umfasst; und
einen am Filter befestigten Filterdraht,
**dadurch gekennzeichnet, dass** der Öffnungsdurchmesser (602) kleiner als der Hauptdurchmesser (601) in einem freien, expandierten Zustand ist, und jedes der Vielzahl von Stützelementen (605) eine Verbiegung (606) bildet, die dem Hauptdurchmesser (601) entspricht, indem diese sich von dem Gewebe (604) nach außen relativ zu einer Mittelachse und zurück zur Mittelachse hin proximal von der Verbiegung (606) zu dem Filterdraht erstreckt.

2. Filteranordnung nach Anspruch 1, ferner umfassend ein röhrenförmiges Element mit einem Lumen.

3. Filteranordnung nach einem der Ansprüche 1 bis 2, wobei das Gewebe eine konische Form umfasst.

4. Filteranordnung nach einem der Ansprüche 1 bis 3, wobei die Stützelemente (605) eine Vielzahl von Drähten (605) umfassen.

5. Filteranordnung nach einem der Ansprüche 1 bis 4, wobei der Filter radial komprimierbar ist.

6. Filteranordnung nach einem der Ansprüche 1 bis 5, wobei der Filter derart konfiguriert ist, dass er in ein Lumen eines Gefäßes in einem komprimierten Zustand passt, und das Gewebe (604) derart konfiguriert ist, dass es mit dem Gefäß im freien, expandierten Zustand in Kontakt steht.

7. Filteranordnung nach einem der Ansprüche 1 bis 6, wobei die Stützelemente (605) derart konfiguriert sind, dass sie mit dem Gefäß im freien, expandierten Zustand in Kontakt stehen.

8. Filteranordnung nach einem der Ansprüche 1 bis 7, wobei der zweite Hauptdurchmesser (602) gleich dem Hauptdurchmesser (601) ist, wenn er sich in einem komprimierten Zustand befindet.

## Revendications

1. Ensemble de filtre (650) comprenant :
un filtre (600) comprenant :
une partie proximale comprenant une pluralité d'éléments de support (605) présentant un diamètre majeur (601), et
une partie distale comprenant une maille (604) présentant un diamètre d'ouverture (602), et
un fil de filtre rattaché au filtre,
**caractérisé en ce que** le diamètre d'ouverture (602) est inférieur au diamètre majeur (601) dans un état libre, expansé, et chacun de la pluralité d'éléments de support (605) forme une courbure (606) correspondant au diamètre majeur (601) en s'étendant de la maille (604) vers l'extérieur par rapport à un axe central et en retournant vers l'axe central dans un sens proximal de la courbure (606) jusqu'au fil de filtre.

2. Ensemble de filtre selon la revendication 1, comprenant en outre un élément tubulaire comportant une lumière.

3. Ensemble de filtre selon l'une quelconque des revendications 1 à 2, dans lequel la maille comprend une forme conique.

4. Ensemble de filtre selon l'une quelconque des revendications 1 à 3, dans lequel les éléments de support (605) comprennent une pluralité de fils (605).

5. Ensemble de filtre selon l'une quelconque des revendications 1 à 4, dans lequel le filtre est radialement compressible.

6. Ensemble de filtre selon l'une quelconque des revendications 1 à 5, dans lequel le filtre est configuré pour s'insérer dans une lumière d'un vaisseau dans un état comprimé, et la maille (604) est configurée pour entrer en contact avec le vaisseau dans l'état libre, expansé.

7. Ensemble de filtre selon l'une quelconque des revendications 1 à 6, dans lequel les éléments de support (605) sont configurés pour entrer en contact avec le vaisseau dans l'état libre, expansé.

8. Ensemble de filtre selon l'une quelconque des revendications 1 à 7, dans lequel le deuxième diamètre majeur (602) est égal au diamètre majeur (601) lorsqu'il est dans un état comprimé.
